(19) Europäisches Patentamt
European Patent Office
Office européen des brevets

(11) Numéro de publication: **0 213 047 B1**

(12) **FASCICULE DE BREVET EUROPÉEN**

(45) Date de publication du fascicule du brevet: **28.02.90**

(51) Int. Cl.[5]: **C 07 C 37/055**, C 07 C 41/26

(21) Numéro de dépôt: **86420195.9**

(22) Date de dépôt: **18.07.86**

(54) **Procédé de préparation de composés aromatiques hydroxylés.**

(30) Priorité: **26.07.85 FR 8511437**

(43) Date de publication de la demande:
**04.03.87 Bulletin 87/10**

(45) Mention de la délivrance du brevet:
**28.02.90 Bulletin 90/09**

(84) Etats contractants désignés:
**AT BE CH DE FR GB IT LI LU NL SE**

(56) Documents cité:
**DE-A-1 257 784**

(73) Titulaire: **RHONE-POULENC CHIMIE**
**25, quai Paul Doumer**
**F-92408 Courbevoie Cédex (FR)**

(72) Inventeur: **Desbois, Michel**
**526, Chemin du Bois**
**F-69140 Rillieux (FR)**

(74) Mandataire: **Le Pennec, Magali**
**RHONE-POULENC INTERSERVICES Service Brevets Chimie 25, Quai Paul Doumer**
**F-92408 Courbevoie Cédex (FR)**

EP 0 213 047 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dipposition (Art. 99(1) Convention sur le brevet européen).

LIBERGRAF, STOCKHOLM 1990

**Description**

La présente invention concerne un procédé de préparation de composés aromatiques hydroxylés. Elle concerne plus particulièrement un procédé de préparation de composés aromatiques hydroxylés et porteurs d'un motif trifluorométhyl.

Il est connu de préparer les composés aromatiques hydroxylés en particulier trifluorométhylés à partir de la trifluorométhylaniline sur laquelle est réalisée une réaction de diazotation (Kirk Othmer, 10, p. 923)

Il est aussi connu d'après le brevet EP 19 388 de préparer des composés aromatiques hydroxylés à partir d'un polyhalogénobenzène qui, dans une première étape, est mis en présence d'un hydroxyde alcalin ou alcalinoterreux dans un mélange de solvant aprotique polaire présentant une constante diélectrique comprise entre 30 et 70 et d'un solvant hydroxylé, le phénate issu de la première étape est mis en contact au cours d'une deuxième étape avec un acide pour former le phénol.

La première méthode permet de synthétiser des métatrifluorométhylphénols.

Le deuxième procédé présente l'inconvénient d'être peu sélectif et donc de donner une grande quantité d'isomères difficilement séparables.

Or, il est toujours plus intéressant de disposer d'une méthode qui puisse sélectivement donner des ortho, méta ou para trifluorométhylphénols.

Dans le document DE-A-1 257 784 est décrit la préparation de phénols porteurs d'un groupe trifluorométhyl ou trifluorométhoxy par un procédé en deux étapes.

La présente invention a permis de trouver un procédé tout à fait nouveau de synthèse de composés aromatiques hydroxylés de formule générale (I)

$Y_n - Ar - (OH)_p$ (I)

dans laquelle Ar représente un radical aromatique mono ou polycyclique éventuellement substitué, Y est un groupe choisi parmi les radicaux trifluorométhyl, trifluorométhoxy, trifluorométhylthio, n est un nombre entier égal à 0, 1 ou 2, p est un nombre entier égal à 1 ou 2 caractérisé en ce qu'on fait réagir dans l'acide fluorhydrique liquide contenant de l'eau un fluoroformiate ou un chloroformiate de formule générale (II)

$Z_n$ - Ar - $(OCOX)_p$ (II)

dans laquelle p et n ont la même signification que précédemment, Z est un groupe choisi parmi les radicaux trihalogénométhyl, trihalogénométhoxy, trihalogénométhylthio, X représente le chlore ou le fluor et que la quantité d'eau est d'au moins une mole par mole de composé de formule (II).

Parmi les substituants éventuels de Ar on peut choisir tous les radicaux ne réagissant pas dans le présent milieu et qui sont connus de tout homme de l'art , on peut citer notamment les radicaux alkyl, alcoxy, halogène, alkylthio, phényl, phénoxy, nitro.

Parmi les composés de formule (II) on préfère utiliser ceux pour lesquels Ar représente un groupe phényl éventuellement substitué.

Parmi les fluoroformiates ou chloroformiates de phényle de formule (II) on préfère utiliser ceux pour lesquels n est égal à 1 et p est égal à 1.

Ainsi il est particulièrement intéressant d'utiliser les fluoroformiates ou chloroformiates de trihalogénométhylphényles, de trihalogénométhoxyphényles, de trihalogénométhylthiophényles.

On préfère utiliser le chloroformiate de trichlorométhylphényle.

On peut citer parmi les composés de formule (II):

- Chloroformiate de chloro-4 phényle
- Chloroformiate de trichlorométhyl-3-phényle
- Chloroformiate de trichlorométhyl-4-phényle
- Chloroformiate de trichlorométhoxy-3 phényle
- Chloroformiate de trichlorométhoxy-4 phényle
- Chloroformiate de trichlorométhyl-4 chloro-2 phényle
- Chloroformiate de trichlorométhyl-4 dichloro-2,6 phényle
- Chloroformiate de trichlorométhoxy-4 chloro-2 phényle
- Chloroformiate de bis (trichlorométhyl)-3,4 phényle
- Chloroformiate de (trichlorométhyl-3 phényl)-4 phényle
- Fluoroformiate de trifluorométhyl-3 phényle
- Fluoroformiate de trifluorométhyl-4 chloro-2 phényle
- Fluoroformiate de trifluorométhoxy-3 phényle
- Fluoroformiate de trifluorométhoxy-4 phényle
- Chloroformiate de trichlorométhylthio-4 phényle
- Chloroformiate de trichlorométhylthio-3 phéyle
- Fluoroformiate de trifluorométhylthio-4 phényle
- Fluoroformiate de trifluorométhylthio-3 phényle
- Dichloroformiate de p-phénylène
- Difluoroformiate de m-phénylène

L'acide fluorhydrique est utilisé selon un rapport molaire acide fluorhydrique au composé de formule (II) compris entre 5 et 50 et de préférence entre 10 et 30.

Il a été découvert de façon tout à fait surprenante que l'addition d'eau accélérait la réaction du fluoroformiate ou du chloroformiate de phényle avec l'acide fluorhydrique. On préfère ajouter une quantité d'eau d'au moins une mole par mole de composé de formule (II) et au maximum de 2 moles par mole de composé de formule (II).

Il faut noter qu'une quantité supérieure n'est pas nuisible au procédé de l'invention mais n'apporte aucun avantage supplémentaire. En effet, il faut ensuite séparer l'eau et l'acide fluorhydrique.

La réaction se déroule notamment sous pression autogène.

La température est de préférence comprise

entre 20 et 150°C et encore plus préférentiellement entre 50 et 100°C.

Lorsque la température est supérieure à 20°C la réaction se déroule de préférence sous pression afin de maintenir l'acide fluorhydrique sous forme liquide.

Les composés de formule (I) issus du présent procédé sont séparés de manière connue en soi, par exemple par distillation de l'acide fluorhydrique présent dans le milieu ou par extraction à l'aide de solvants organiques.

Les composés issus de la présente invention sont utilisés comme intermédiaires de synthèse dans l'industrie pharmaceutique ou phytosanitaire (EP 54149 et EP 49383).

Les exemples suivants sont donnés à titre illustratifs mais ne doivent pas être considérés comme limitatifs de l'invention.

**Exemple 1 – m – Trifluoromethylphenol**

Dans un réacteur inox de 250 ml agité par un barreau aimanté, on introduit successivement 100 g (5 m) d'acide fluorhydrique anhydre 54,8 g (0,2 m) de chloroformiate de m-trichlorométhylphényle et 7,2 g (0,4 m) d'eau. Le réacteur est fermé puis porté progressivement à la température de 80°C pendant un temps total de 3h 50. Après refroidissement aux environs de 0°C et décompression, le brut acide réactionnel est coulé sur 200 g de glace pilée. Le mélange hétérogène ainsi obtenu est extrait par 3 fois 100 $cm^3$ de $CH_2Cl_2$, les phases organiques sont rassemblées, lavées par 2 fois 100 $cm^3$ d'eau puis séchées. Après évaporation on récupère 20 g d'un composé essentiellement constitué de m-trifluorométhylphénol (Analysés par infrarouge, chromatographie en phase gazeuse et spectrométrie de masse).

**Exemple 2 – m – Trifluoromethylphenol**

On procède de manière identique à l'exemple 1 avec les conditions et produits suivants:

- Acide fluorhydrique anhydre: 100 g – 5 m
- Chloroformiate de m-trichlorométhylphényle: 82,2 g – 0,3 m
- Eau: 8,1 g – 0,45 m
- Température: 80°C
- Durée: 4h 50

Après traitements et analyses (infrarouge, chromatographie en phase gazeuse et spectrométrie de masse) on récupère 39 g de m-trifluorométhylphénol brut.

**Exemple 3 – m –Trifluoromethylphenol**

On procède de manière identique à l'exemple 1 avec les conditions et produits suivants:

- Acide fluorhydrique anhydre:50 g – 2,5 m
- Chloroformiate de m-trichlorométhylphényle: 41,1 g – 0,15 m
- Eau: 3,6 g – 0,2 m
- Température:60°C
- Durée: 2 heures

Après traitements et analyses (infrarouge, chromatographie en phase gazeuse et spectrométrie de masse) on récupère 19 g de m-trifluorométhylphénol brut.

**Exemple 4 – p – Trifluoromethylphenol**

On procède de manière identique à l'exemple 1 avec les conditions et produits suivants :

- Acide fluorhydrique anhydre:100 g – 5 m
- Chloroformiate de p-trichlorométhylphényle: 54,8 g – 0,2 m
- Eau: 5,4 g – 0,3 m
- Température: 60°C
- Durée: 4h 30

Après traitements et analyses (infrarouge, chromatographie en phase gazeuse et spectrométrie de masse) on récupère 20 g de p-trifluorométhylphénol brut.

**Exemple 5 – p – Chlorophenol**

On procède de manière identique à l'exemple 1 avec les conditions et produits suivants:

- Acide fluorhydrique anhydre: 60 g – 3 m
- Chloroformiate de p-chlorophényle: 38,2 g – 0,2 m
- Eau: 4,5 g – 0,25 m
- Température: 80°C
- Durée: 4 heures

Après traitements et analyses (infrarouge, chromatographie en phase gazeuse et spectrométrie de masse) on récupère 21 g de p-chlorophénol brut.

**Exemple 6 – p – Trifluoromethoxyphenol**

On procède de manière identique à l'exemple 1 avec les conditions et produits suivants:

- Acide fluorhydrique anhydre: 60 g – 3 m
- Chloroformiate de p-trichlorométhoxyphényle: 43,5 g – 0,15 m
- Eau: 3,6 g – 0,2 m
- Température: 120°C
- Durée: 3 heures

Après traitements et analyses (infrarouge, chromatographie en phase gazeuse et spectrométrie de masse) on récupère 18 g de p-trifluorométhoxyphénol brut.

**Revendications**

1. Procédé de préparation de composés aromatiques hydroxylés de formule générale (I)

$Y_n - Ar - (OH)_p$ (I)

dans laquelle Ar représente un radical aromatique mono ou polycyclique éventuellement substitué, Y est un groupe choisi parmi les radicaux trifluorométhyl, trifluorométhoxy, trifluorométhylthio, n est un nombre entier égal à 0, 1 ou 2, p est un nombre entier égal à 1 ou 2 caractérisé en ce qu'on fait réagir dans l'acide fluorhydrique liquide, contenant de l'eau un fluoroformiate ou un chloroformiate de formule générale (II)

$Z_n - Ar - (OCOX)_p$ (II)

dans laquelle p et n ont la même signification que précédemment, Z est un groupe choisi parmi les radicaux trihalogénométhyl, trihalogénométhoxy, trihalogénométhylthio, X représente le chlore ou le fluor et que la quantité d'eau est d'au moins une mole par mole de composé de formule (II).

2. Procédé selon l'une quelconque des revendications précédentes caractérisé en ce que dans le composé de formule (II) Ar représente un groupe phényl éventuellement substitué.

3. Procédé selon la revendication 1 caractérisé en ce que n est égal à 1 et p est égal à 1.

4. Procédé selon l'une quelconque des revendications précédentes caractérisé en ce que Ar représente le groupe phényl non substitué.

5. Procédé selon l'une quelconque des revendications précédentes caractérisé en ce que dans la formule (II) Z est un groupe trihalogénométhyl.

6. Procédé selon l'une quelconque des revendications précédentes caractérisé en ce que Z est un groupe trichlorométhyl.

7. Procédé selon l'une quelconque des revendications précédentes caractérisé en ce que le rapport molaire de l'acide fluorhydrique au composé de formule (II) est compris entre 5 et 50 et de préférence entre 10 et 20.

8. Procédé selon l'une quelconque des revendications précédentes caractérisé en ce que l'on ajoute une quantité d'au moins une mole d'eau par mole de composé de formule (II) et d'au maximum 2 moles d'eau par mole de composé de formule (II).

**Claims**

1. Process for preparing hydroxylated aromatic compounds of general formula (I)

$Y_n - Ar - (OH)_p$ (I)

in which Ar denotes an optionally substituted mono- or polycyclic aromatic radical, Y is a group chosen from trifluoromethyl, trifluoromethoxy and trifluoromethylthio radicals, n is an integer equal to 0, 1 or 2, and p is an integer equal to 1 or 2, characterized in that a fluoroformate or chloroformate of general formula (II)

$Z_n - Ar - (OCOX)_p$ (II)

in which p and n have the same meaning as above, Z is a group chosen from trihalomethyl, trihalomethoxy and trihalomethylthio radicals and X denotes chlorine or fluorine, is reacted in liquid hydrofluoric acid containing water, and in that the quantity of water is at least one mole per mole of compound of formula (II).

2. Process according to any one of the preceding claims, characterized in that, in the compound of formula (II), Ar denotes an optionally substituted phenyl group.

3. Process according to claim 1, characterized in that n equals 1 and p equals 1.

4. Process according to any one of the preceding claims, characterized in that Ar denotes an unsubstituted phenyl group.

5. Process according to any one of the preceding claims, characterized in that, in the formula (II), Z is a trihalomethyl group.

6. Process according to any one of the preceding claims, characterized in that Z is a trichloromethyl group.

7. Process according to any one of the preceding claims, characterized in that the mole ratio of hydrofluoric acid to the compound of formula (II) is between 5 and 50, and preferably between 10 and 20.

8. Process according to any one of the preceding claims, characterized in that an amount of at least one mole of water is added per mole of compound of formula (II) and at most 2 moles of water per mole of compound of formula (II).

**Patentansprüche**

1. Verfahren zur Herstellung von aromatischen Hydroxylverbindungen der allgemeinen Formel (I)

$Y_n - Ar - (OH)_p$ (I),

in der Ar einen aromatischen, mono- oder polycyclischen, gegebenenfalls substituierten Ring bedeutet, Y eine Gruppe ist, ausgewählt aus Trifluormethyl, Trifluormethoxy, Trifluormethylthio, n eine ganze Zahl gleich 0, 1 oder 2 ist und p eine

ganze Zahl gleich 1 oder 2 bedeutet, dadurch gekennzeichnet, daß man in flüssiger, Wasser enthaltender Fluorwasserstoffsäure ein Fluorformiat oder ein Chlorformiat der allgemeinen Formel (II)

$Z_n - Ar - (OCOX)_p$ (II),

in der p und n die oben angegebene Bedeutung haben, Z eine Gruppe ist, ausgewählt aus Trihalogenmethyl, Trihalogenmethoxy, Trihalogenmethylthio, und X Chlor oder Fluor bedeutet, zur Reaktion bringt und daß die Menge an Wasser mindestens 1 mol pro mol Verbindung der Formel (II) beträgt.

2. Verfahren nach einem der vorangehenden Ansprüche, dadurch gekennzeichnet, daß bei der Verbindung der Formel (II) Ar eine gegebenenfalls substituierte Phenylgruppe bedeutet.

3. Verfahren nach Anspruch 1 dadurch gekennzeichnet, daß n gleich 1 und p gleich 1 ist.

4. Verfahren nach einem der vorangehenden Ansprüche, dadurch gekennzeichnet, daß Ar eine nicht substituierte Phenylgruppe bedeutet.

5. Verfahren nach einem der vorangehenden Ansprüche, dadurch gekennzeichnet, daß in der Formel (II) Z eine Trihalogenmethylgruppe ist.

6. Verfahren nach einem der vorangehenden Ansprüche, dadurch gekennzeichnet, daß Z eine Trichlormethylgruppe ist.

7. Verfahren nach einem der vorangehenden Ansprüche, dadurch gekennzeichnet, daß das Molverhältnis von Fluorwasserstoffsäure zu Verbindung der Formel (II) zwischen 5 und 50, vorzugsweise zwischen 10 und 20, liegt.

8. Verfahren nach einem der vorangehenden Ansprüche, dadurch gekennzeichnet, daß man eine Menge von mindestens 1 mol Wasser pro mol Verbindung der Formel (II) und höchstens 2 mol Wasser pro mol Verbindung der Formel (II) zusetzt.